# EUROPEAN PATENT APPLICATION

(11) **EP 4 559 685 A1**
(43) Date of publication of application: **28.05.2025**
(21) Application number: 24214066.3
(22) Date of filing: 20.11.2024
(51) Int. Cl.: B41J 2/175, B41J 2/185, B41J 2/195, B41J 29/17, B41J 11/00, G01N 21/47, G01N 21/85, G01N 33/32, B41J 2/21

(54) **WASHING DEVICE AND RECORDING DEVICE**

(30) Priority: 24.11.2023 JP 2023198913
(71) Applicant: Seiko Epson Corporation, Tokyo 160-8801 (JP)
(72) Inventor: ONISHI, Yasunori, Suwa-shi, Nagano, 392-8502 (JP); MATSUZAKI, Takahiro, Suwa-shi, Nagano, 392-8502 (JP); NISHIZAWA, Shuhei, Suwa-shi, Nagano, 392-8502 (JP); HIYAMA, Sota, Suwa-shi, Nagano, 392-8502 (JP)
(74) Representative: MERH-IP Matias Erny Reichl Hoffmann Patentanwälte PartG mbB

(57) **Abstract**

The washing device includes a storage section (35) that stores washing liquid for washing a transport belt that transports a medium, an optical sensor (53) that detects ink density of dye ink that was mixed in the washing liquid stored in the storage section, and a control section that performs control so as to cause the washing liquid to be discharged from the storage section. The optical sensor has a light emitting section (61) that emits light and a light receiving section (62) that receives the light emitted from the light emitting section. Based on a detection result by the optical sensor, the control section performs control to discharge the washing liquid from the storage section. The light emitted from the light emitting section includes light whose wavelength is less than or equal to 400 nm.

## Description

The present application is based on, and claims priority from JP Application Serial Number 2023-198913, filed November 24, 2023, the disclosure of which is hereby incorporated by reference herein in its entirety.

### BACKGROUND

### 1. Technical Field

The present disclosure relates to a washing device and a recording device.

### 2. Related Art

JP-A-2008-213394 describes a washing device that washes a transport belt, which transports a medium, with washing liquid. In the washing device, washing the transport belt causes ink to mix in the washing liquid. The washing device detects density of ink that has been mixed in the washing liquid based on an amount of light transmitted through the washing liquid. If the ink density is greater, the washing device discharges the washing liquid.

However, for inks with no particles present in the washing liquid due to dissolution in the washing liquid, it was not easy for such a washing device to accurately detect the ink density based on the amount of light transmitted through the washing liquid. As described above, it is desired to appropriately discharge the washing liquid by accurately detecting the ink density mixed in the washing liquid.

### SUMMARY

In order to solve the above problem, a washing device includes a storage section that stores washing liquid for washing a transport belt that transports a medium; an optical sensor that detects ink density of dye ink that was mixed in the washing liquid stored in the storage section, and a control section that performs control so as to cause the washing liquid to be discharged from the storage section, wherein the optical sensor has a light emitting section that is configured to emit light and a light receiving section that is configured to receive the light emitted from the light emitting section, the control section performs control, based on a detection result by the optical sensor, such that the washing liquid is discharged from the storage section, and the light emitted from the light emitting section includes light whose wavelength is less than or equal to 400 nm.

In order to solve the above problem, a recording device including the above washing device, a transport section that has a transport belt, and a recording section that performs recording by ejecting dye ink onto a medium transported by the transport section.

### BRIEF DESCRIPTION OF THE DRAWINGS

FIG. 1 is a schematic view showing a recording device provided with a washing device according to a first embodiment.
FIG. 2 Is a schematic diagram showing a detection section in the first embodiment.
FIG. 3 is a graph showing a relationship between wavelength of light and transmissivity of dye ink in the first embodiment.
FIG. 4 is another graph showing the relationship between the wavelength of light and the transmissivity of dye ink in the first embodiment.
FIG. 5 is a graph showing a relationship between ink density and output level in the first embodiment.
FIG. 6 is another graph showing the relationship between the ink density and the output level in the first embodiment.
FIG. 7 is a graph showing a relationship between transmittance of dye ink and coefficient of determination, and a relationship between the transmittance of dye ink and a slope of the ink density of dye ink in the first embodiment.
FIG. 8 is a graph showing the relationship between the ink density and the output level in the first embodiment.

### DESCRIPTION OF EMBODIMENTS

### FIRST EMBODIMENT

Hereinafter, an embodiment of a recording device including a washing device will be described.

### Configuration of recording device 11

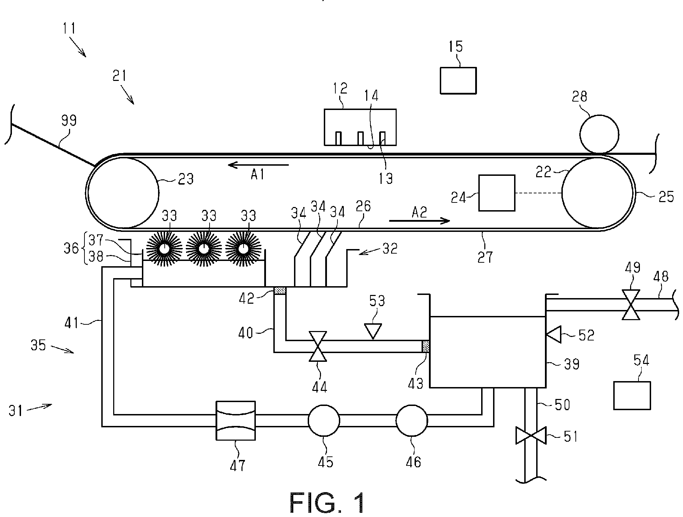

As shown in FIG. 1, the recording device 11 is an inkjet type printer that records an image by ejecting liquid onto a medium. The medium may be, for example, fabric, paper, or the like. The image may be text, a photograph, or the like. The liquid may be, for example, dye ink. The liquid may include reactive dye ink. The liquid may include, for example, acid dye ink. In other words, the liquid may include the reactive dye ink or the acid dye ink.

The dye ink is ink containing dye in solvent. The dye ink includes ink in which particles are not present in the washing liquid due to dissolution in the washing liquid. The dye ink may include, for example, black, cyan, yellow, magenta, red, orange, green, light black, and the like inks.

### Configuration of recording section 12

The recording device 11 is equipped with a recording section 12. The recording section 12 is configured to eject liquid onto a medium 99. The recording section 12 is configured to eject liquid onto the medium 99 transported by a transport section 21, which will be described in detail later. By this, the recording section 12 records an image on the medium 99. The recording section 12 is a head. The recording section 12 has a nozzle surface 14 in which one or more nozzles 13 are opened. The recording section 12 ejects liquid from the nozzles 13. The recording section 12 may be a serial head that scans the medium 99. The recording section 12 may be a line head capable of ejecting liquid simultaneously over the width of the medium 99.

### Configuration of the transport section 21

The recording device 11 is equipped with a transport section 21. The transport section 21 is configured to transport the medium 99. In a process in which the transport section 21 transports the medium 99, the liquid is ejected from the recording section 12 to the medium 99.

The transport section 21 is configured to transport the medium 99 in the transport direction A1. The transport section 21 includes a plurality of transport rollers. For example, the transport section 21 includes a first transport roller 22 and a second transport roller 23. The first transport roller 22 is located upstream of the recording section 12 in the transport direction A1. The second transport roller 23 is located downstream of the recording section 12 in the transport direction A1.

The transport section 21 has a drive source 24. The drive source 24 is, for example, a motor. The drive source 24 is connected to at least one transport roller of the plurality of transport rollers. In one example, the drive source 24 is connected to the first transport roller 22. The drive source 24 rotates the first transport roller 22.

The transport section 21 has a transport belt 25. The transport belt 25 is wound around a plurality of transport rollers. In one example, the transport belt 25 is wound around the first transport roller 22 and the second transport roller 23. The transport belt 25 rotates along the first transport roller 22 and the second transport roller 23 by the drive source 24 rotating the first transport roller 22. In FIG. 1, the transport belt 25 rotates counterclockwise. By this, the transport belt 25 transports the medium 99. The transport belt 25 supports the medium 99 on which an image is recorded by the recording section 12.

The transport belt 25 includes a portion that moves in the transport direction A1 and a portion that moves in an opposite direction A2, which is opposite to the transport direction A1. The transport belt 25 transports the medium 99 by the portion moving in the transport direction A1. The portion moving in the opposite direction A2 is washed by a washing device 31 (to be described later).

The transport belt 25 has an inner circumferential surface 26 and an outer circumferential surface 27. The inner circumferential surface 26 is a surface that comes into contact with a plurality of transport rollers. In one example, the inner circumferential surface 26 is in contact with the first transport roller 22 and the second transport roller 23. The outer circumferential surface 27 is a surface that comes into contact with the medium 99. The outer circumferential surface 27 is a surface that supports the medium 99. The outer circumferential surface 27 faces the nozzle surface 14.

The transport belt 25 is configured so that the medium 99 can be adhere to the belt. Specifically, a medium 99 is adhered to the outer circumferential surface 27. Thus, the medium 99 is transported in a stable posture. For example, the transport belt 25 is an adhesive belt on which an adhesive is applied. The adhesive is applied to the outer circumferential surface 27. In this case, the medium 99 is adhered to the outer circumferential surface 27 by the adhesive. Not limited to adhesive, the medium 99 may be adhered to the outer circumferential surface 27 by, for example, suction force, electrostatic force, intermolecular force, or the like. After an image is recorded on the medium 99 by the recording section 12, the medium 99 is separated from the outer circumferential surface 27, for example, by being pulled by another device. The transport section 21 may have a pull-off section that pulls the medium 99 away from the outer circumferential surface 27. In this case, the pull-off section is, for example, a roller around which the medium 99 is wound.

The transport section 21 may have a pressing section 28. The pressing section 28 is configured to press the medium 99 against the transport belt 25. In one example, the pressing section 28 is a roller. The pressing section 28 presses the medium 99 against the transport belt 25, which adheres the medium 99 to the outer circumferential surface 27.

In the transport belt 25, recording of an image on the medium 99 by the recording section 12 may cause dye ink ejected as liquid to adhere to the outer circumferential surface 27. If the transport belt 25 continues the transportation of the medium 99 in a state in which the dye ink adheres to the outer circumferential surface 27, the medium 99 may become dirty.

### Configuration of recording control section 15

The recording device 11 is equipped with a recording control section 15. The recording control section 15 may control the recording section 12. The recording control section 15 may control the transport section 21. The recording control section 15 may be composed of one or a plurality of processors. The processor executes various processes according to a computer program. The recording control section 15 may be composed of one or more dedicated hardware circuitries. The recording control section 15 may include an application specific integrated circuit that executes at least a part of various processes. The recording control section 15 may be composed of a processor and a circuit including a combination of hardware circuitries. The processor includes a CPU and a memory, such as a RAM and a ROM. The memory stores program code or commands that are configured to cause the CPU to perform processes. The Memory, that is, a computer readable medium, includes any readable medium that can be accessed by a general-purpose or dedicated computer.

### Configuration of washing device 31

The recording device 11 is equipped with a washing device 31. The washing device 31 is configured to wash the transport belt 25 with washing liquid. Specifically, the washing device 31 washes the outer circumferential surface 27 with the washing liquid. The washing device 31 removes dye ink adhering to the outer circumferential surface 27 by the washing liquid. The washing liquid makes it easier for dye ink to be removed from transport belt 25. The washing liquid is liquid for washing the transport belt 25. In one example, the washing liquid is water.

### Configuration of the washing section 32

The washing device 31 has a washing section 32. The washing section 32 washes the transport belt 25 with the washing liquid. The washing section 32 washes the transport belt 25 by contacting the transport belt 25. Specifically, the washing section 32 comes into contact with the outer circumferential surface 27. The washing section 32 comes into contact with a portion of the transport belt 25 which is located in the opposite direction A2. In other words, the washing section 32 comes into contact with the transport belt 25 from which the medium 99 has been pulled off.

The washing section 32 has one or more washing rollers 33. In one example, the washing section 32 has three washing rollers 33. The washing rollers 33 come into contact with the transport belt 25 while rotating. The washing rollers 33 may be driven to rotate with respect to the transport belt 25. In other words, the washing rollers 33 may be driven to rotate clockwise in FIG. 1. The washing rollers 33 may be driven and rotate so as to resist against the rotation of the transport belt 25. In other words, the washing rollers 33 may be rotated counterclockwise in FIG. 1. In this case, the contact resistance between the washing rollers 33 and the transport belt 25 is increased. Therefore, dye ink is easier to be removed from the transport belt 25.

The washing rollers 33 come into contact with the transport belt 25 in a state of being wetted with the washing liquid. By this, the transport belt 25 is wetted with the washing liquid. Therefore, dye ink is easier to be removed from the transport belt 25. The washing rollers 33 are, for example, brush rollers. The washing rollers 33 may be sponge rollers.

The washing section 32 has one or more washing blades 34. In one example, the washing section 32 has three washing blades 34. The washing blades 34 come into contact with the transport belt 25, which is wet with the washing liquid. The washing blades 34 come into contact with the outer circumferential surface 27, which is wet with the washing liquid. The washing blades 34 scrape off dye ink together with the washing liquid adhering to the transport belt 25. The washing blades 34 come into contact with the transport belt 25 after the washing rollers 33 came into contact with the transport belt 25. Therefore, the washing blades 34 are located in the opposite direction A2 side from the washing rollers 33.

### Configuration of storage section 35

The washing device 31 is equipped with a storage section 35. The storage section 35 is configured to store the washing liquid. The storage section 35 holds the washing section 32. The storage section 35 is configured so as to store the washing liquid that is supplied to the washing section 32. The washing section 32 washes the transport belt 25 using the washing liquid stored in the storage section 35. The washing liquid stored in the storage section 35 gradually becomes dirty as the washing section 32 washes the transport belt 25. In other words, dye ink adhered to the transport belt 25 may be mixed in the washing liquid that is stored in the storage section 35. By this, the storage section 35 may store the washing liquid in which the dye ink is mixed. Therefore, the washing device 31 needs to appropriately change the washing liquid stored in the storage section 35.

The storage section 35 has a washing tank 36. The washing tank 36 is a tank that stores the washing liquid. The washing tank 36 is located so as to face the outer circumferential surface 27. The washing tank 36 holds the washing section 32. As the washing rollers 33 wash the transport belt 25, the dye ink flows into the washing tank 36. In other words, the washing tank 36 stores the washing liquid after it has been used by the washing section 32.

The washing tank 36 has an immersing tank 37. The immersing tank 37 is a tank that stores a predetermined amount of washing liquid. An immersing tank 37 holds the washing rollers 33. The immersing tank 37 holds the washing rollers 33 so that the washing rollers 33 are partially immersed in the stored washing liquid. In other words, the washing tank 36 is a tank in which the washing section 32 is immersed in the stored washing liquid. As the washing rollers 33 wash the transport belt 25, dye ink flows into the immersing tank 37. In other words, the immersing tank 37 stores the washing liquid after it has been used by the washing section 32.

The washing tank 36 has a support tank 38. The support tank 38 supports the immersing tank 37. Specifically, the support tank 38 accommodates the immersing tank 37. The support tank 38 receives washing liquid that overflows from the immersing tank 37.

The support tank 38 supports the washing blades 34. The support tank 38 receives washing liquid flowing down along the washing blades 34. As the washing blades 34 wash the transport belt 25, dye ink flows into the support tank 38.

The storage section 35 has a container tank 39. The container tank 39 is a tank that is connected to the washing tank 36. The container tank 39 is a tank that collects washing liquid from the washing tank 36. The container tank 39 may be a tank that circulates the washing liquid stored in the washing tank 36. The container tank 39 stores washing liquid that has been used by the washing section 32. The container tank 39 may store the washing liquid that has been used by the washing section 32.

The storage section 35 has a connection flow path. The storage section 35 may have a plurality of connection flow paths. In one example, the storage section 35 has a first connection flow path 40 and a second connection flow path 41. The first connection flow path 40 and the second connection flow path 41 are connection flow paths that are connected to the washing tank 36 and the container tank 39. Specifically, the first connection flow path 40 is connected to the support tank 38 and the container tank 39. The second connection flow path 41 is connected to the immersing tank 37 and the container tank 39.

Washing liquid flows from the washing tank 36 to the container tank 39 through the first connection flow path 40. Specifically, the washing liquid flows from the support tank 38 to the container tank 39 through the first connection flow path 40. Washing liquid flows from the container tank 39 to the washing tank 36 through the second connection flow path 41. Specifically, the washing liquid flows from the container tank 39 to the immersing tank 37 through the second connection flow path 41. In this way, the washing liquid is circulated between the washing tank 36 and the container tank 39 in the storage section 35. By this, density of the dye ink included in the washing liquid tends to be more uniform.

The washing device 31 may be equipped with one or more filters. The washing device 31 is equipped with a washing filter 42 and a container filter 43. The filter collects dust, fluff, and the like in the washing liquid. The washing filter 42 is attached to the first connection flow path 40. Specifically, the washing filter 42 is located at the end section of the first connection flow path 40 that is connected to the washing tank 36. The washing filter 42 collects foreign matters from the washing liquid flowing from the washing tank 36 to the container tank 39. The container filter 43 is attached to the first connection flow path 40. Specifically, the container filter 43 is located at an end section that is connected to the container tank 39 in the first connection flow path 40. The container filter 43 collects foreign matters from the washing liquid flowing from washing tank 36 to container tank 39.

The washing device 31 is equipped with a first opening and closing valve 44. The first opening and closing valve 44 is, for example, a solenoid valve. The first opening and closing valve 44 is located in the first connection flow path 40. By opening the first opening and closing valve 44, the washing liquid can flow from the washing tank 36 to the container tank 39.

The washing device 31 is equipped with a pump 45. The pump 45 circulates the washing liquid in the storage section 35. The pump 45 circulates the washing liquid between the washing tank 36 and the container tank 39 through the first connection flow path 40 and the second connection flow path 41. In this way, the container tank 39, the second connection flow path 41, and the pump 45 function as an example of the circulation section. In one example, the pump 45 is located in the second connection flow path 41. The pump 45 may be located in the first connection flow path 40.

The washing device 31 is equipped with a flow amount meter 46. The flow amount meter 46 measures a flow mount of the washing liquid flowing in the storage section 35. The flow amount meter 46 measures a flow amount of the washing liquid that is circulating between the washing tank 36 and the container tank 39. The washing device 31 controls the pump 45 based on a measurement result of the flow amount meter 46. In one example, the flow amount meter 46 is located in the second connection flow path 41. Specifically, the flow amount meter 46 is located between the pump 45 and the container tank 39 in the second connection flow path 41. The flow amount meter 46 may be located in the first connection flow path 40.

The washing device 31 is equipped with a constant flow amount valve 47. The constant flow amount valve 47 is configured to maintain a flow amount of the washing liquid flowing in the storage section 35 at a constant flow amount. The constant flow amount valve 47 is a so-called throttle valve. The constant flow amount valve 47 maintains a flow amount of the washing liquid circulating between the washing tank 36 and the container tank 39 at a constant flow amount. In one example, the constant flow amount valve 47 is located in the second connection flow path 41. Specifically, the constant flow amount valve 47 is located between the pump 45 and the washing tank 36 in the second connection flow path 41. The constant flow amount valve 47 may be located in the first connection flow path 40.

The washing device 31 is equipped with a supply flow path 48. The supply flow path 48 is a flow path through which the washing liquid is supplied. A new washing liquid is supplied to the washing device 31 through the supply flow path 48. The supply flow path 48 is connected to the storage section 35. In one example, the supply flow path 48 is connected to the container tank 39. The supply flow path 48 is connected to, for example, a water supply. When the washing liquid in the storage section 35 is reduced due to evaporation of the washing liquid, discharge of the washing liquid, or the like, the washing liquid is supplied from the supply flow path 48 to the storage section 35.

The washing device 31 is equipped with a second opening and closing valve 49. The second opening and closing valve 49 is, for example, a solenoid valve. The second opening and closing valve 49 is located in the supply flow path 48. When the second opening and closing valve 49 is opened, the washing liquid is supplied to the storage section 35.

The washing device 31 is equipped with a discharge flow path 50. The discharge flow path 50 is a flow path through which the washing liquid is discharged. The washing liquid is discharged from the washing device 31 through the discharge flow path 50. In one example, the discharge flow path 50 is connected to the container tank 39. The discharge flow path 50 is connected to, for example, a treatment device that treats waste liquid, a waste liquid tank that stores waste liquid, and the like. When washing liquid is contaminated by dye ink, the washing liquid is discharged from storage section 35 through the discharge flow path 50.

The washing device 31 is equipped with a third opening and closing valve 51. The third opening and closing valve 51 is, for example, a solenoid valve. The third opening and closing valve 51 is located in the discharge flow path 50. When the third opening and closing valve 51 is opened, the washing liquid is discharged from the storage section 35.

The washing device 31 may be equipped with a liquid amount sensor 52. The liquid amount sensor 52 is a sensor that measures an amount of washing liquid that is stored in the storage section 35. In one example, the liquid amount sensor 52 measures the amount of the washing liquid accommodated in the container tank 39. The washing device 31 can maintain, by the liquid amount sensor 52, the amount of washing liquid stored in the storage section 35 at a constant amount. For example, when the amount of the washing liquid stored in the container tank 39 is large, the washing device 31 discharges the washing liquid through the discharge flow path 50. When the amount of the washing liquid stored in the container tank 39 is small, the washing device 31 supplies the washing liquid through the supply flow path 48.

The washing device 31 is equipped with a detection section 53. The detection section 53 is configured to detect ink density of the dye ink that was mixed in the washing liquid stored in the storage section 35. The detection section 53 is provided in the first connection flow path 40. The detection section 53 may be provided in the second connection flow path 41. In other words, the detection section 53 is provided in the connection flow path. Hereinafter, the ink density of the dye ink mixed in the washing liquid that is stored in storage section 35 is simply referred to as ink density of dye ink.

The washing device 31 is equipped with a washing control section 54. The washing control section 54 controls the washing device 31. The washing control section 54 receives output results from the flow amount meter 46, the liquid amount sensor 52, and the detection section 53. The washing control section 54 controls the first opening and closing valve 44, the pump 45, the constant flow amount valve 47, the second opening and closing valve 49, and the third opening and closing valve 51.

Similar to the recording control section 15, the washing control section 54 may be composed of a processor, may be composed of a hardware circuitry, or may be composed of a circuit including a combination thereof. The washing control section 54 corresponds to an example of a control section. The washing device 31 may be controlled by the recording control section 15. In such a case, the recording control section 15 corresponds to an example of the control section.

The washing control section 54 controls so that the washing liquid is discharged from the storage section 35. The washing control section 54 performs control to cause the washing liquid to be discharged from the storage section 35 by opening the first opening and closing valve 44 and the third opening and closing valve 51.

In particular, the washing control section 54 performs control, based on a detection result by the detection section 53, such that the washing liquid is discharged from the storage section 35. The washing control section 54 discharges the washing liquid when the ink density of dye ink included in the washing liquid that is stored in the storage section 35 is greater. If the ink density of dye ink included in the liquid stored in storage section 35 becomes greater, the washing capacity of the washing device 31 decreases. By discharging the washing liquid when the ink density of dye ink included in the liquid stored in the storage section 35 is greater, usage amount of the washing liquid is reduced compared to a configuration in which the washing liquid is constantly being supplied.

### Configuration of detection section 53

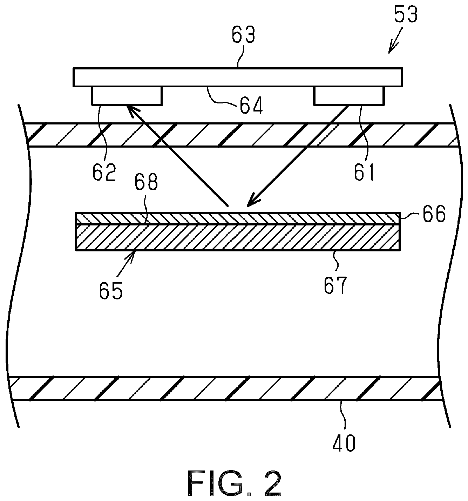

Here, a configuration of detection section 53 will be described with reference to FIG. 2. As shown in FIG. 2, detection section 53 includes an optical sensor. In other words, the washing device 31 is equipped with the optical sensor. The detection section 53 is equipped with a light emitting section 61 and a light receiving section 62. In other words, the optical sensor is equipped with the light emitting section 61 and the light receiving section 62. The detection section 53 may be equipped with a substrate 63. The substrate 63 has an installation surface 64. The installation surface 64 is a surface that faces toward the first connection flow path 40. The light emitting section 61 and the light receiving section 62 are installed on the installation surface 64. In other words, the light emitting section 61 and the light receiving section 62 are provided so as to face in the same direction.

The light emitting section 61 includes a light emitting element. The light emitting section 61 includes, for example, a LED. The light emitting section 61 emits light of a predetermined wavelength. As described above, the light emitting section 61 is configured to emit light.

The light receiving section 62 includes an element that outputs a signal by receiving light. The light receiving section 62 includes, for example, a photodiode. In this way, the light receiving section 62 is configured to receive the light that is emitted from the light emitting section 61.

The first connection flow path 40 is equipped with a reflection section 65. In other words, the washing device 31 is equipped with the reflection section 65. The reflection section 65 is provided inside the first connection flow path 40. The reflection section 65 is provided at a position that faces the detection section 53. The reflection section 65 is provided at a position that faces the installation surface 64. In other words, the reflection section 65 is provided at a position that faces the light emitting section 61 and the light receiving section 62. The reflection section 65 is configured to reflect light.

The first connection flow path 40 has at least translucency between the light emitting section 61 and the light receiving section 62 and the reflection section 65. By this, the reflection section 65 is configured to reflect the light from the light emitting section 61 to the light receiving section 62. The light emitting section 61 emits light toward the first connection flow path 40. That light is reflected by the reflection section 65 and then enters the light receiving section 62. By this, the detection section 53 can detect the transmitted light.

The reflection section 65 may be equipped with a reflection sheet 66 and an installation member 67. The reflection sheet 66 is a sheet that reflects light. The reflection sheet 66 is attached to the installation member 67. The reflection sheet 66 is provided on a facing surface 68 of the installation member 67. The facing surface 68 is a surface that faces the detection section 53. In other words, the reflection sheet 66 is provided on the detection section 53 side surface of the installation member 67.

The reflection sheet 66 is provided at a position at a predetermined distance from the detection section 53. The predetermined distance may be a distance determined so as to sufficiently reflect the light from the light emitting section 61 to the light receiving section 62. The predetermined distance may be a distance determined according to a type of detection section 53.

By the reflection section 65, the light emitting section 61 and the light receiving section 62 may not be provided at opposite positions that faces each other over the first connection flow path 40. In other words, the light emitting section 61 and the light receiving section 62 may not be provided at positions sandwiching the first connection flow path 40. In one example, the light emitting section 61 and the light receiving section 62 are arranged along the first connection flow path 40. In this way, the degree of freedom regarding the positions of the light emitting section 61 and the light receiving section 62 is improved by the reflection section 65.

In the storage section 35, dye that constitutes the dye ink is dissolved in the washing liquid. The detection section 53 detects the ink density of dye ink by detecting the dye that is dissolved in the washing liquid. The detection section 53 optically detects the dye included in the washing liquid.

As a method for optically detecting the dye included in the washing liquid, there is a transmission method. The transmission method is a method that detects dye based on an amount of transmitted light that passes through the washing liquid. In other words, the transmission method is a method that uses a property of dye to absorb light.

The light receiving section 62 receives the light from the light emitting section 61. The light receiving section 62 receives the light from the light emitting section 61 by the reflection of the reflection section 65. The light receiving section 62 receives the light from the light emitting section 61 through the liquid in the first connection flow path 40.

The detection section 53 can detect ink density of dye ink using the liquid stored in the first connection flow path 40 as an evaluation target. In other words, the detection section 53 detects the ink density of dye ink in the liquid that is being flowing through the first connection flow path 40.

The detection section 53 detects the ink density of dye ink using light whose wavelength is less than or equal to 400 nm. In particular, the detection section 53 desirably uses light whose wavelength is between 340 nm and 380 nm, and it is optimal to use light whose wavelength is 360 nm.

As described above, the light emitted from the light emitting section 61 includes light whose wavelength is less than or equal to 400 nm, desirably includes light whose wavelength is greater than or equal to 340 nm and less than or equal to 380 nm, and optimally includes light whose wavelength is 360 nm.

The detection section 53 may be equipped with an optical filter. The optical filter is a filter that blocks light in a predetermined frequency band. As a specific example, the optical filter may be a filter that transmits light whose wavelength is 400 nm or less and blocks light whose wavelength is greater than 400 nm. The optical filter may be a filter that transmits light whose wavelength is between 340 nm and 380 nm and that blocks light of other wavelengths.

The optical filter may be disposed so as to block light in a predetermined frequency band of the light that is received by the light receiving section 62. In such a case, the light receiving section 62 may be configured to be able to receive light whose wavelength is less than or equal to 400 nm. In other words, the light receiving section 62 may be configured to receive at least light whose wavelength is less than or equal to 400 nm.

The optical filter may be disposed so as to block light in a predetermined frequency band of light that is emitted from the light emitting section 61. In such a case, the light emitting section 61 may be configured to emit light whose wavelength is less than or equal to 400 nm. In other words, the light emitting section 61 may be configured to emit light whose wavelength is at least 400 nm or less.

### Characteristic of dye ink

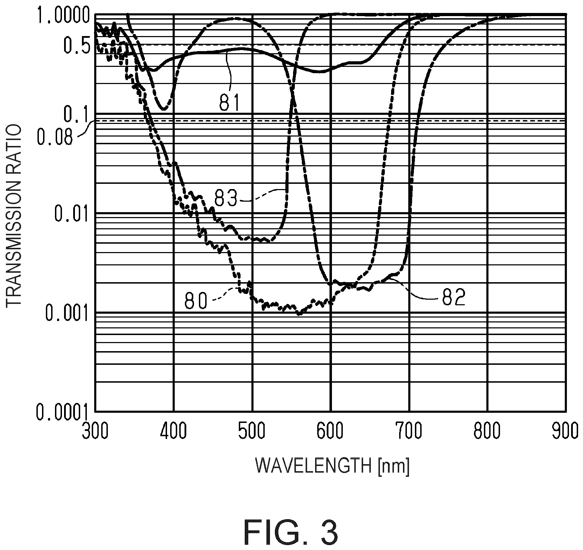

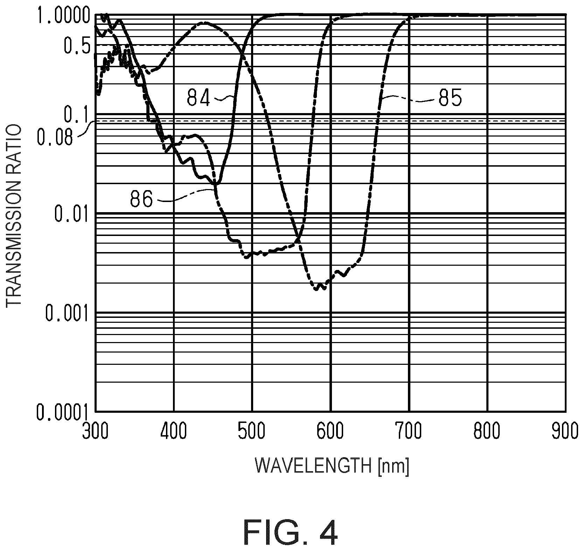

Next, characteristics of the dye ink will be described with reference to FIGS. 3 to 8. FIGS. 3 and 4 are graphs showing a relationship between wavelength of light and transmissivity of dye ink. In particular, FIGS. 3 and 4 are graphs showing transmissivity of liquid, which contains a single color reactive dye ink and a washing liquid, when light is irradiated to the liquid. Black, gray, cyan, orange, yellow, red, and blue inks are examples of the single color reactive dye ink. Graphs 80 to 83 in FIG. 3, in order, are graphs showing black single color, gray single color, cyan single color, and orange single color reactive dye inks as evaluation targets. Graphs 84 to 86 in FIG. 4, in order, are graphs showing yellow single color, red single color, and blue single color reactive dye inks as evaluation targets. The evaluation targets are considered to be liquid whose ink density of single color reactive dye ink is 0.5%.

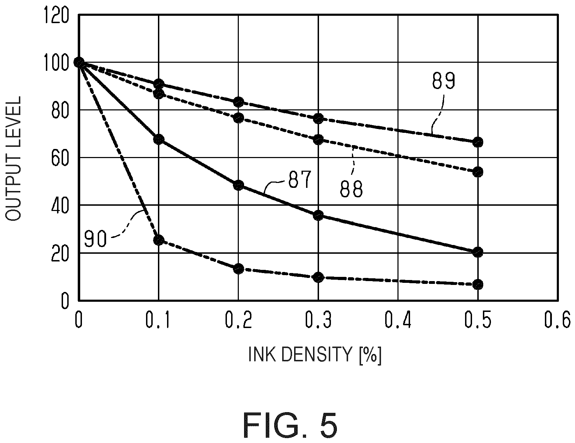

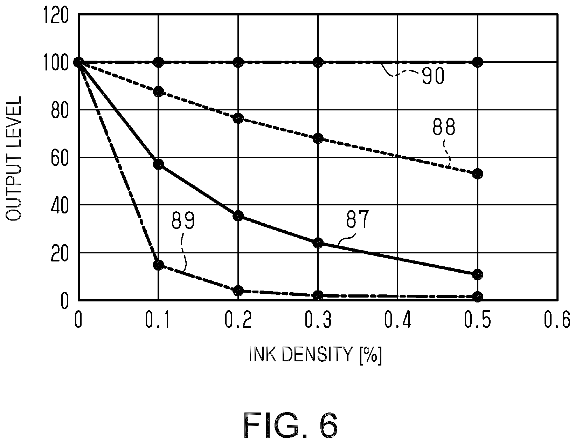

FIGS. 5 and 6 are graphs showing a relationship between ink density and output level for each of single color reactive dye inks. The output level is a level indicated by an amount of light received by the light receiving section 62. Examples of single color reactive dye inks include blue, gray, cyan and orange inks. Graphs 87 to 90 in FIGS. 5 and 6, in order, are graphs showing blue single color, a gray single color, a cyan single color, and an orange single color reactive dye inks as evaluation targets. FIG. 5 is a graph showing the output levels when irradiated with light whose wavelength is 525 nm. FIG. 6 is a graph showing the output levels when irradiated with light whose wavelength is 660 nm.

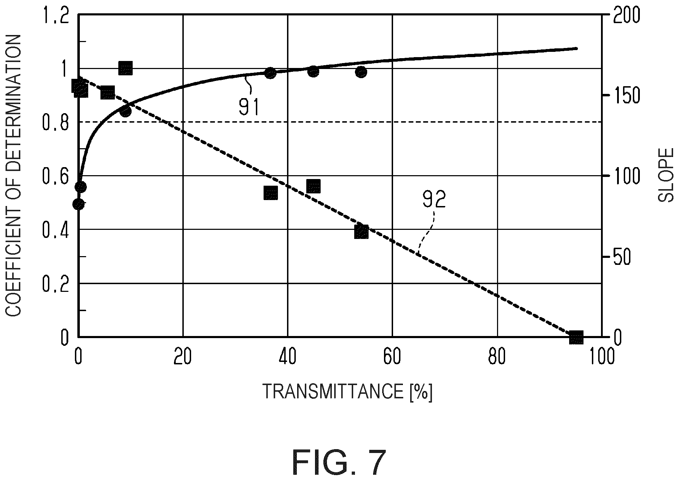

FIG. 7 is a graph showing a relationship between transmittance of reactive dye ink and coefficient of determination, and a relationship between the transmittance of reactive dye ink and slope of the ink density of reactive dye ink. The slope of the ink density of reactive dye ink indicates a slope between the ink density of reactive dye ink and an output level. Graph 91 in FIG. 7 is a graph showing a relationship between the transmittance of reactive dye ink and the coefficient of determination. Graph 92 in FIG. 7 is a graph showing a relationship between the transmittance of reactive dye ink and the slope of the ink density of reactive dye ink.

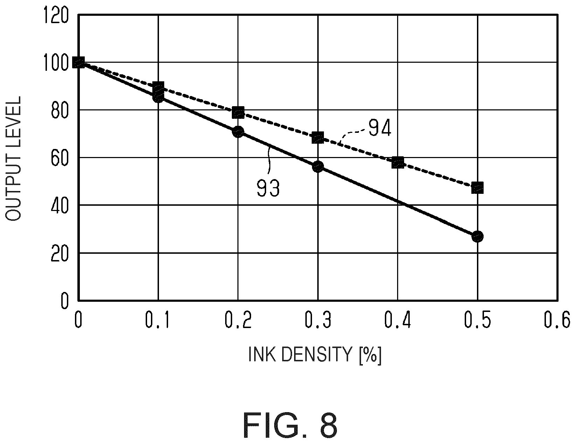

FIG. 8 is a graph showing a relationship between the ink density and the output level. FIG. 8 is a graph when the coefficient of determination is set to 1. Graph 93 in FIG. 8 is a graph in which a black single color reactive dye ink is set as the evaluation target. Graph 94 in FIG. 8 is a graph in which a cyan single color reactive dye ink is set as the evaluation target.

As shown in FIG. 3 and FIG. 4, transmissivity of the reactive dye inks with respect to the wavelength of light differs depending on a color of the reactive dye ink. In other words, the dye inks have different light absorbance with respect to the wavelength of light, depending on the color of the dye ink.

As shown in FIGS. 5 and 6, in the reactive dye inks, the ink density of reactive dye ink is inversely proportional to the output level. In other words, the greater the ink density of reactive dye ink is, the smaller the output level becomes. As described above, the greater the ink density of dye ink is, the smaller the amount of light received by the light receiving section 62 becomes.

In particular, the greater the ink density of reactive dye ink is, the greater linearity of the graph becomes. The higher the linearity of the graph is, the smaller an error in a relationship between the ink density of dye ink and the output level becomes. Therefore, the higher the linearity of the graph is, the more accurate the detection of the ink density of dye ink based on the output level becomes.

The output level with respect to the ink density of reactive dye ink varies depending on color of the reactive dye ink. If light of a different wavelength is irradiated to the liquid including reactive dye ink and washing agent, the output level with respect to the ink density of reactive dye ink will change.

As shown in FIG. 7, an appropriate condition for improving the detection accuracy of the ink density is to satisfy both a first reference and a second reference. The first reference is a reference that the coefficient of determination is greater than or equal to 0.8. The second reference is a reference that a slope of the ink density is greater than or equal to 80. When the ink density is 0.5%, the second reference corresponds to a slope of the ink density at which the output level decreases by 40%. In such a case, ink transmittance of 8% to 50% will be the appropriate condition. In other words, 0.08 to 0.5 will become the appropriate condition for the ink transmission ratio.

As shown in FIGS. 3 and 4, light of wavelength that is greater than or equal to 340 nm and less than or equal to 380 nm tends to have less variation in the ink transmission ratio for each color ink. Therefore, it is optimal that the light emitted by the light emitting section 61 includes light whose wavelength is greater than or equal to 340 nm and less than or equal to 380 nm. The light emitted from the light emitting section 61 may include ultraviolet light, such as light with a wavelength of 400 nm or less. The light emitted from the light emitting section 61 may include violet visible light, such as light whose wavelength is greater than or equal to 380 nm and less than or equal to 430 nm.

As shown in FIG. 8, when a light with a wavelength of 360 nm is used, the linearity of both graphs 93 and 94 becomes higher. Graph 93 indicates the black single color reactive dye ink as an example that tends to have a low transmittance. Graph 94 indicates the cyan single color reactive dye ink as an example that tends to have a low transmittance. As described above, the linearity of the graph becomes high in the dye ink of each color.

Therefore, the detection section 53 detects the amount of light received by the light receiving section 62 using light with a wavelength of 400 nm or less. By this, it is possible to detect the ink density of dye ink. In particular, it is optimum that the detection section 53 uses light with a wavelength of 360 nm, and it is preferable that the detection section 53 uses light with a wavelength of 340 nm to 380 nm, which is an acceptable range centered at the wavelength of 360 nm.

### Control by the washing control section 54

The washing control section 54 determines whether or not to discharge washing liquid by comparing a detection result of the detection section 53 with a threshold value. When the ink density of dye ink is greater than or equal to the threshold value, the washing control section 54 discharges washing liquid from the storage section 35. At this time, the washing control section 54 opens the first opening and closing valve 44 and the third opening and closing valve 51, then discharges the washing liquid from the storage section 35. After completing discharge of the washing liquid, the washing control section 54 closes the third opening and closing valve 51 and opens the second opening and closing valve 49 to supply washing liquid to the storage section 35. By this, the washing liquid is exchanged.

When discharging the washing liquid, the washing control section 54 may determine whether or not the recording device 11 is recording. The washing control section 54 may determine, by communicating with the recording control section 15, whether or not the recording device 11 is being recording. The washing control section 54 may determine, by accepting an operation from the user, whether or not the recording device 11 is being recording. In a case where the recording device 11 is performing recording, it is desirable that washing liquid is stored in the washing tank 36. Therefore, in a case where the recording device 11 is performing recording, the washing control section 54 discharges washing liquid from the container tank 39 by opening the second opening and closing valve 49 while the first opening and closing valve 44 is closed. By this, while washing liquid is held in the washing tank 36, washing liquid is exchanged in the container tank 39.

### Operation and Effect of First Embodiment

The operations and effects of the first embodiment will be described.
(1) The washing control section 54 controls to discharge washing liquid from the storage section 35 based on a detection result by the detection section 53. The light emitted from the light emitting section 61 includes light whose wavelength is less than or equal to 400 nm. According to this configuration, it is possible to improve the detection accuracy of the ink density of dye ink that was mixed in the washing liquid by using light with a wavelength that satisfies an appropriate condition even if dye ink is a target ink. Therefore, by accurately detecting the ink density of ink mixed in the washing liquid, the washing liquid can be appropriately discharged.
(2) The wavelength of light emitted from the light emitting section 61 includes light whose wavelength is greater than or equal to 340 nm and less than or equal to 380 nm. According to this configuration, it is possible to further improve the detection accuracy of the ink density of dye ink that was mixed in the washing liquid by using light with a wavelength that satisfies an optimal condition even if dye ink is a target. Therefore, by accurately detecting the ink density of ink mixed in the washing liquid, the washing liquid can be appropriately discharged.
(3) The washing device 31 may be equipped with the reflection section 65 provided on the facing surface 68 that faces the installation surface 64 on which the light emitting section 61 and the light receiving section 62 are installed. According to this configuration, it is possible to adjust an arrangement of the light emitting section 61 and the light receiving section 62 by the reflection section 65. By this, it is possible to further improve the detection accuracy of the ink density of dye ink that was mixed in the washing liquid even if dye ink is a target. Therefore, by accurately detecting the ink density mixed in the washing liquid, the washing liquid can be appropriately discharged.
(4) The light from the light emitting section 61 can be received by the light receiving section 62 even if the light emitting section 61 and the light receiving section 62 are not facing each other. By this, the light emitting section 61 and the light receiving section 62 can be collectively provided. Therefore, the washing device 31 can be reduced in size.
(5) The storage section 35 includes the washing tank 36, the container tank 39, and the first connection flow path 40. The washing tank 36 is a tank in which the washing section 32 is immersed in the stored washing liquid. The first connection flow path 40 is provided so as to connect the washing tank 36 and the container tank 39. The detection section 53 is provided in the first connection flow path 40. According to this configuration, the ink density of dye ink can be detected in the first connection flow path 40, which tends to have a smaller diameter than the washing tank 36 and the container tank 39. By this, it is possible to further improve the detection accuracy of the ink density of dye ink that was mixed in the washing liquid even if dye ink is a target. Therefore, by accurately detecting the ink density mixed in the washing liquid, the washing liquid can be appropriately discharged.
(6) The washing device 31 is equipped with the pump 45 that circulates washing liquid between the washing tank 36 and the container tank 39 through the first connection flow path 40 and the second connection flow path 41. According to this configuration, by circulating the washing liquid between the washing tank 36 and the container tank 39, the ink density of dye ink contained in the washing liquid is more likely to be uniform. By this, it is possible to further improve the detection accuracy of the ink density of dye ink that was mixed in the washing liquid even if dye ink is a target. Therefore, by accurately detecting the ink density mixed in the washing liquid, the washing liquid can be appropriately discharged.

### Example of modification

The present embodiment can be implemented with the following modifications. The present embodiment and the following modification examples can be implemented in combination with each other within a technically compatible range.
- The detection section 53 may be provided in the storage section 35. The detection section 53 may be provided in the container tank 39. The detection section 53 may be provided in the washing tank 36. In this case, it is possible to detect the ink density of dye ink in the washing tank 36 in which the washing section 32 is immersed in the washing liquid. By this, it is possible to further improve the detection accuracy of the ink density of dye ink that was mixed in the washing liquid even if dye ink is a target. Therefore, by accurately detecting the ink density mixed in the washing liquid, the washing liquid can be appropriately discharged.
- If the detection section 53 is provided in the washing tank 36, the washing device 31 may not need to have the container tank 39. In this case, the washing device 31 may be configured such that the discharge flow path 50 is directly connected to the washing tank 36. By this, the washing device 31 can configure the storage section 35 with a minimum functionality. Therefore, the washing device 31 can be reduced in size and cost.
- The reflection section 65 may not be configured to attach the reflection sheet 66 to the installation member 67 as long as it can reflect light from the light emitting section 61 to the light receiving section 62. As long as the reflection section 65 can reflect light from the light emitting section 61 to the light receiving section 62, the reflection sheet 66 and the installation member 67 may be integrally configured.
- In the region where the detection section 53 is provided, the diameter of the first connection flow path 40 may be reduced. The light emitting section 61 and the light receiving section 62 may be located at positions sandwiching the first connection flow path 40. In this case, the washing device 31 may not need to have the reflection section 65.
- As an object to which the disclosure is applied, not only the washing device 31 but also the recording device 11 having the function of the washing device 31 can be used. The recording device 11 may have some of functions of the washing device 31, or may have all of the functions of the washing device 31.
- As used herein, the phrase "at least any" means one or more of the desired options. As an example, the phrase "at least any" as used herein means only one option or both of the two options if the number of options is two. As another example, the expression "at least any" as used herein means only one option or a combination of any two or more options if the number of options is three or more.

### Notes

Hereinafter, technical ideas grasped from the above-described embodiment and modifications, and operations and effects thereof will be described. The present technical idea and the operations and effects thereof can be combined with each other within a technically consistent range.
(A) The washing device includes a storage section that stores washing liquid for washing a transport belt that transports a medium; an optical sensor that detects ink density of dye ink that was mixed in the washing liquid stored in the storage section, and a control section that performs control so as to cause the washing liquid to be discharged from the storage section, wherein the optical sensor has a light emitting section that is configured to emit light and a light receiving section that is configured to receive the light emitted from the light emitting section, the control section performs control, based on a detection result by the optical sensor, such that the washing liquid is discharged from the storage section, and the light emitted from the light emitting section includes light whose wavelength is less than or equal to 400 nm.
   According to this configuration, it is possible to improve the detection accuracy of the ink density of dye ink that was mixed in the washing liquid by using light with a wavelength that satisfies an appropriate condition even if dye ink is a target ink. Therefore, by accurately detecting the ink density of ink mixed in the washing liquid, the washing liquid can be appropriately discharged.
(B) According to the above washing device, wavelength of light emitted from the light emitting section may include light whose wavelength is greater than or equal to 340 nm and less than or equal to 380 nm.
   According to this configuration, it is possible to further improve the detection accuracy of the ink density of dye ink that was mixed in the washing liquid by using light with a wavelength that satisfies an optimal condition even if dye ink is a target. Therefore, by accurately detecting the ink density of ink mixed in the washing liquid, the washing liquid can be appropriately discharged.
(C) The above washing device may be further equipped with a reflection section provided on a facing surface that faces an installation surface on which the light emitting section and the light receiving section are installed.
   According to this configuration, the arrangement of the light emitting section and the light receiving section can be adjusted by the reflection section. By this, it is possible to further improve the detection accuracy of the ink density of dye ink that was mixed in the washing liquid even if dye ink is a target. Therefore, by accurately detecting the ink density mixed in the washing liquid, the washing liquid can be appropriately discharged.
   Further, without facing the light emitting section and the light receiving section each other, the light receiving section can receive light from the light emitting section. By this, the light emitting section and the light receiving section can be collectively provided. Therefore, the washing device can be reduced in size.
(D) The above washing device may further include a washing section that washes the transport belt with the washing liquid, wherein the storage section includes a washing tank, a container tank, and a connection flow path, the washing tank is a tank in which the washing section is partially immersed in the washing liquid stored in the washing tank, the connection flow path is provided to connect the washing tank and the container tank, and the optical sensor is provided in the connection flow path.
   According to this configuration, the ink density of dye ink can be detected in the connection flow path that tends to have a smaller diameter than the washing tank and the container tank. By this, it is possible to further improve the detection accuracy of the ink density of dye ink that was mixed in the washing liquid even if dye ink is a target. Therefore, by accurately detecting the ink density mixed in the washing liquid, the washing liquid can be appropriately discharged.
(E) The above washing device may further include a washing section that washes the transport belt with the washing liquid, wherein the storage section includes a washing tank that stores washing liquid, the washing tank is a tank in which the washing section is partially immersed in the washing liquid stored in the washing tank, the optical sensor is provided in the washing tank.
   According to this configuration, the optical sensor can be provided in the washing tank in which the washing section is immersed in the washing liquid. By this, it is possible to further improve the detection accuracy of the ink density of dye ink that was mixed in the washing liquid even if dye ink is a target. Therefore, by accurately detecting the ink density mixed in the washing liquid, the washing liquid can be appropriately discharged.
(F) A recording device includes the above washing device, a transport section that has a transport belt, and a recording section that performs recording by ejecting dye ink onto a medium transported by the transport section. According to this configuration, the same effect as (A) can be achieved.

## Claims

1. A washing device, comprising:
a storage section that stores washing liquid for washing a transport belt that transports a medium;
an optical sensor that detects ink density of dye ink that was mixed in the washing liquid stored in the storage section, and
a control section that performs control so as to cause the washing liquid to be discharged from the storage section, wherein
the optical sensor has a light emitting section that is configured to emit light and a light receiving section that is configured to receive the light emitted from the light emitting section,
the control section performs control, based on a detection result by the optical sensor, such that the washing liquid is discharged from the storage section, and
the light emitted from the light emitting section includes light whose wavelength is less than or equal to 400 nm.

2. The washing device according to claim 1, wherein
the wavelength of the light emitted from the light emitting section includes light whose wavelength is greater than or equal to 340 nm and less than or equal to 380 nm.

3. The washing device according to claim 1, further comprising:
a reflection section provided on a facing surface that faces an installation surface where the light emitting section and the light receiving section are installed.

4. The washing device according to claim 1, further comprising:
a washing section that washes the transport belt with the washing liquid, wherein
the storage section includes a washing tank, a container tank, and a connection flow path,
the washing tank is a tank in which the washing section is partially immersed in the washing liquid stored in the washing tank,
the connection flow path is provided to connect the washing tank and the container tank, and
the optical sensor is provided in the connection flow path.

5. The washing device according to claim 1, further comprising:
a washing section that washes the transport belt with the washing liquid, wherein
the storage section includes a washing tank that stores washing liquid,
the washing tank is a tank in which the washing section is partially immersed in the washing liquid stored in the washing tank, and
the optical sensor is provided in the washing tank.

6. A recording device comprising:
a washing device according to claim 1,
a transport section that has a transport belt, and
a recording section that performs recording by ejecting dye ink onto a medium transported by the transport section.
